# EUROPÄISCHE PATENTSCHRIFT

(11) **EP 0 301 298 B1**
(45) Veröffentlichungstag und Bekanntmachung des Hinweises auf die Patenterteilung: **17.03.1993**
(21) Anmeldenummer: 88111020.9
(22) Anmeldetag: 11.07.1988
(51) Int. Cl.: C07H 15/04

(54) **Verfahren zur Herstellung von Alkylglykosiden**
Process for the preparation of alkyl glycosides
Procédé de préparation de glucosides d'alkyle

(30) Priorität: 18.07.1987 DE 3723826
(43) Veröffentlichungstag der Anmeldung: 01.02.1989
(73) Patentinhaber: Henkel Kommanditgesellschaft auf Aktien, 40191 Düsseldorf (DE)
(72) Erfinder: Biermann, Manfred, Dr., D-4330 Mülheim (DE); Hill, Karlheinz, Dr., D-4006 Erkrath (DE); Wüst, Willi, Dr., D-4030 Ratingen (DE); Eskuchen, Rainer, Dr., D-4000 Düsseldorf-Benrath (DE); Wollmann, Josef, D-5120 Herzogenrath 3 (DE); Bruns, Andreas, Dr., D-4018 Langenfeld (DE); Hellmann, Günter, Dr., D-4010 Hilden (DE); Ott, Karl-Heinz, Dr., D-4006 Erkrath (DE); Winkle, Walter, Dr., D-4019 Monheim (DE); Wollmann, Klaus, Dr., D-5657 Haan (DE)

(56) Entgegenhaltungen:
- EP-A- 0 092 875
- EP-A- 0 132 046
- US-A- 3 974 138

## Beschreibung

Bei der Entwicklung neuer oberflächenaktiver Stoffe, die als industrielle Tenside für die Herstellung von Wasch- und Reinigungsmitteln geeignet sind, werden zur Herstellung in zunehmendem Maße nachwachsende Rohstoffe verwendet. Dafür kommen bisher hauptsächlich fettchemische Rohstoffe, wie z. B. Fettsäuren, Fettsäureester und Fettalkohole in Betracht. Ziel dieser Entwicklungen ist es, eine von der Petrochemie unabhängige Basis auszubauen und damit aber auch zu besseren umweltverträglichen Produkten, die biologisch gut abbaubar sind, zu gelangen. Unter diesen Gesichtspunkten sind neuerdings die oberflächenaktiven Alkylglykoside, bei denen es sich um Acetale aus Zuckern und Fettalkoholen handelt, interessant geworden.

Im folgenden werden unter dem Begriff Alkylglykoside die Reaktionsprodukte aus Zuckern und Fettalkoholen verstanden, wobei als Zuckerkomponente die im folgenden als Glykosen bezeichneten Aldosen bzw. Ketosen Glucose, Fructose, Mannose, Galactose, Talose, Gulose, Allose, Altrose, Idose, Arabinose, Xylose, Lyxose und Ribose in Betracht kommen. Die besonders bevorzugten Alkylglykoside sind wegen der leichten Zugänglichkeit der Glucose die Alkylglucoside. Der Begriff Alkyl in Alkylglykosid soll im weitesten Sinne den Rest eines aliphatischen Alkohols, der den hydrophoben Teil in das oberflächenaktive Alkylglykosid einbringt, d. h. einen Rest mit wenigstens 8 Kohlenstoffatomen,vorzugsweise den Rest eines primären aliphatischen Alkohols, und insbesondere den Rest eines Fettalkohols erhältlich aus natürlichen Fetten, also gesättigte und ungesättigte Reste und deren Gemische einschließlich solcher mit verschiedenen Kettenlängen, umfassen. Die Begriffe Alkyloligoglykosid, Alkylpolyglykosid, Alkyloligosaccharid und Alkylpolysaccharid beziehen sich auf solche alkylierten Glykosen, in denen 1 Alkylrest in Form des Acetals an mehr als einen Glykoserest, also an einen Poly- oder Oligosaccharidrest gebunden ist; diese Begriffe werden als untereinander gleichbedeutend angesehen. Entsprechend bedeutet Alkylmonoglykosid das Acetal eines Monosaccharids. Da die Reaktionsprodukte aus den Zuckern und den primären Alkoholen in der Regel Gemische darstellen, werden unter dem Begriff Alkylglykosid sowohl Alkylmonoglykoside als auch Alkylpoly(oligo)glykoside verstanden, sofern es nicht ausdrücklich auf die strukturellen Unterschiede ankommt.

Die oberflächenaktiven Alkylglykoside mit im wesentlichen C₁₂- bis C₁₈-Alkyl- bzw. Alkenyl-Resten gehören zum Typ der nichtionischen Tenside. Während aber bei den üblichen nichtionischen Tensiden vom Typ der Alkylpolyglykolether allenfalls der hydrophobe Teil von nachwachsenden Rohstoffen stammt, wenn er sich von Fettalkoholen ableitet, und der hydrophile Teil aus Ethylenoxideinheiten und damit aus einem petrochemischen Rohstoff aufgebaut ist, können die Alkylglykoside als Fettalkylglykoside vollständig aus nachwachsenden Rohstoffen, nämlich Fett einerseits und Zuckern bzw. Stärken andererseits hergestellt werden.Die oberflächenaktiven Alkylglykoside sind als Waschmittelrohstoffe bereits seit über 50 Jahren bekannt. So beschreibt die österreichische Patentschrift Nr. 135 333 die Herstellung von Laurylglucosid und Cetylglucosid aus Acetobromglucose und dem jeweiligen Fettalkohol in Gegenwart einer Base. Aber auch die Direktsynthese aus Glucose und Laurylalkohol mit Chlorwasserstoff als saurem Katalysator wird erwähnt. Nach der Lehre der deutschen Patentschrift 611 055 werden Alkylglucoside aus Pentaacetylglucose und dem Fettalkohol in Gegenwart von wasserfreiem Zinkchlorid hergestellt. Aus der deutschen Patentschrift Nr. 593 422 sind die Maltoside und Lactoside der aliphatischen Alkohole mit mehr als 8 Kohlenstoffatomen und ihre Verwendung als Emulgierungs-, Reinigungs- und Netzmittel bekannt. Beispielsweise wird durch Zusatz von Cetylmaltosid zu gewöhnlicher Seife, die damals der Hauptbestandteil der Waschmittel war, der Wascheffekt der Seife verbessert, was mit der Wirkung des Cetylmaltosids als Kalkseifendispergator erklärt wird. Aus den 60er und 70er Jahren stammen mehrere Vorschläge zu verbesserten Herstellungsverfahren für Alkylglykoside entweder durch direkte Umsetzung der Glykose, meist in Form von Glucose, mit einem Überschuß des Fettalkohols und einer Säure als Katalysator (Direktsynthese), oder unter Mitverwendung eines niederen Alkohols oder Glykols als Lösungsmittel und Reaktionspartner (Umacetalisierung). So wird in der US-Patentschrift 3,547,828 (Mansfield et al) die Herstellung eines ternären Gemisches aus Alkyloligoglucosiden, Alkylmonoglucosiden und den entsprechenden C₁₁-C₃₂-Alkanolen nach dem Umacetalisierungsverfahren mit Butanol beschrieben. Dabei wird zunächst die Glucose mit Butanol und einem saurem Katalysator, z. B. Schwefelsäure, zu Butylglucosid umgesetzt, wobei das Reaktionswasser bei der Rückflußtemperatur abgeschieden wird. Man benutzt dabei 2 bis 6 Mol Butanol pro Mol Glucose. Danach wird der Fettalkohol in Mengen von 0,5 bis 4 Mol pro Mol Glucose hinzugegeben und das überschüssige Butanol sowie das bei der Umacetalisierungsreaktion gebildete Butanol abdestilliert. Die Umacetalisierungsreaktion wird gegebenenfalls abgebrochen, so daß noch Teile des Butylglucosids im Reaktionsgemisch verbleiben. Auf diese Weise lassen sich Produkte mit niedriger Viskosität herstellen. Anschließend wird der saure Katalysator durch Zugabe von Natriumhydroxidlösung neutralisiert. Danach wird im Vakuum der überschüssige Fettalkohol zum größten Teil bis auf das gewünschte Niveau entfernt, meist auf Werte von weniger als 2 Gew.-%. Z. B. läßt sich so Laurylalkohol bei 150 °C und 2 mm Hg Vakuum abdestillieren. Aus dem so erhaltenen Dreiergemisch aus Alkylmonoglucosid, Alkyloligoglucosid und Fettalkohol, gegebenenfalls mit Anteilen an Butylglucosid, läßt sich der Anteil an Alkyloligoglucosid durch Behandeln mit Aceton, in dem die Oligoglucoside unlöslich sind, isolieren. Nach einem Vorschlag der US-Patentschrift 3,450,690 (Gibbons et al) werden unerwünschte alkaliempfindliche, Verfärbungen hervorrufende Verunreinigungen von Alkylglucosiden auf Basis von C₁-C₈-Alkanolen, hergestellt nach der Methode der Direktsynthese, dadurch aus dem Reaktionsprodukt entfernt, daß man durch Zusatz von anorganischen oder organischen Basen, wie z. B. Natriumhydroxid, Natriummethylat, Calciumhydroxid, Bariumhydroxid, Bariummethylat oder stark basischen Aminen den sauren Katalysator nicht nur neutralisiert, sondern einen alkalischen pH-Wert von wenigstens 8 einstellt und dann auf Temperaturen zwischen 50 °C und 200 °C kurzzeitig erhitzt. Danach wird abfiltriert und der Alkohol-Überschuß entfernt. Durch diese Behandlung sollen die restlichen Mengen an reduzierendem Zucker, die für die Farbinstabilität des unbehandelten Produkts verantwortlich gemacht werden, entfernt werden.

Ein weiteres Problem, das bei der Herstellung der oberflächenaktiven Alkylglykoside auf Basis der üblichen Fettalkohole mit 12 bis 18 Kohlenstoffatomen auftritt, liegt in der Schwierigkeit der destillativen Abtrennung des nichtumgesetzten Anteils dieser Fettalkohole aus dem Reaktionsprodukt. Dazu wird in der europäischen Patentanmeldung 32 252 (BASF, Klahr et al) vorgeschlagen, die destillative Abtrennung dieser nichtumgesetzten Fettalkohole in Gegenwart von solchen Glykolen durchzuführen, deren Siedepunkte die der abzutrennenden Alkohole um höchstens 10 °C über- und um höchstens 30 °C unterschreiten. Auf diese Weise kann die Destillation bei nicht mehr als 140 °C und einem Vakuum von etwa 8 mbar, d. h. in einer für das Produkt schonenden Weise durchgeführt werden.

Nach der Lehre der europäischen Patentanmeldung 92 875 (Procter & Gamble, Mao) wird das Umacetalisierungsverfahren mit Butanol zur Herstellung von langkettigen Alkylglucosiden so gesteuert, daß noch eine Restmenge an Butylglucosiden von weniger als 10 Gew.-% im Verfahrensprodukt enthalten sind. Auf diese Weise wird die Bildung der langkettigen Alkyloligoglucoside mit höherem Oligomerisierungsgrad, d. h. mit 6 und mehr Glucoseeinheiten im Molekül verringert. Die so erhaltenen Produkte bestehen im wesentlichen aus Alkylmonoglucosid und Alkyloligoglucosiden, wobei der Mengenanteil der Alkylmonoglucoside maximal 60 Gew.-% und der durchschnittliche Oligomerisierungsgrad 1,5 bis 3 beträgt. Der Anteil an kurzkettigen Alkylglucosiden, insbesondere Butylglucosiden, liegt unterhalb 10 %, der Anteil an nichtumgesetztem Fettalkohol soll unter 2 % liegen. Zur destillativen Entfernung des Fettalkohols wird die Benutzung eines Dünnschichtverdampfers empfohlen.

Die europäische Patentanmeldung 92 876 (Procter & Gamble, Mao et al) beschreibt ebenfalls die Herstellung von langkettigen Alkylglucosiden mit einem Oligomerisierungsgrad von 1,5 bis 20 nach dem Umacetalisierungsverfahren mit Butanol, wobei der die Umacetalisierung bewirkende Katalysator (Paratoluolsulfonsäure) durch Neutralisation dann inaktiviert wird, wenn wenigstens 90 % des Butylglucosids reagiert haben, so daß noch höchstens 10 % Butylglucosid im Reaktionsprodukt verbleiben. Zur schonenden Entfernung des Fettalkoholüberschusses wird hier ebenfalls die Verwendung eines Dünnschichtverdampfers empfohlen. Die Reaktionsprodukte sollen ebenfalls weniger als 60 Gew.-% Alkylmonoglucosid und weniger als 2 % freien Fettalkohol enthalten. Bei diesem bekannten Herstellungsverfahren wird ein möglichst geringer Überschuß an Fettalkohol verwendet, damit möglichst große Mengen des gewünschten Alkyloligoglucosids, d. h. möglichst in Mengen von mehr als 60 Gew.-%, anfallen.

Die europäische Patentanmeldung 96 917 (Procter & Gamble, Farris) beschreibt ein verbessertes Verfahren zu der säurekatalysierten Direktsynthese, wobei ein Monosaccharid, vorzugsweise Glucose, kontinuierlich oder portionsweise so zu der Mischung aus Fettalkohol und Katalysator bei 80 bis 150 °C hinzugefügt wird, daß nie mehr als 10 % nichtumgesetztes Monosaccharid im Reaktionsgemisch vorhanden sind. Die Monosaccharid-Zugabe wird so gesteuert, daß ständig eine im wesentlichen klare Phase vorliegt.

Vorzugsweise wird das Monosaccharid in fein vermahlenem Zustand in Mischung mit einem Teil des Fettalkohols eingesetzt. Gleichzeitig mit der Monosaccharid-Zugabe wird das entstehende Wasser abdestilliert, wobei man einen Unterdruck von 0,1 bis 300 mm Hg anwendet. Nach dem üblichen Aufarbeiten liefert das Verfahren ein Produkt, das 20 bis 70 % Alkylmonoglykosid, weniger als 10 % Mono- und Polysaccharide, weniger als 2 % freien Fettalkohol, und im übrigen Alkylpolyglykoside, d. h. im wesentlichen Di-, Tri- und Tetraglykoside enthält.

In der europäischen Patentanmeldung 132 046 (Procter & Gamble, Letton) wird zur Neutralisation des sauren Katalysators bei einem Verfahren der Direktsynthese eine organische Base, die entweder das Alkali(Na,K,Li)-, Erdalkali(Ba,Ca)- oder Aluminiumsalz einer schwachen niedermolekularen Säure, z. B. Natriumacetat, oder das entsprechende Alkoholat, z. B. Natriumethylat, darstellt, verwendet. Dabei wird ein enger pH-Wert-Bereich um den Neutralpunkt (pH 6,6 bis 7, vorzugsweise 6,7 bis 6,8) eingestellt.

In der europäischen Patentanmeldung 132 043 (Procter & Gamble, Davis et al) wird als saurer Katalysator die Säureform eines anionischen Tensids verwendet, durch dessen Verwendung anstelle üblicher Katalysatoren wie Schwefelsäure oder Paratoluolsulfonsäure, die Farbqualität des Produkts verbessert und ein Gehalt des Endprodukts an dem unerwünschten Polysaccharid herabgesetzt werden soll. Bei dem Verfahren dieser Literaturstelle werden vorzugsweise 2 Mol Fettalkohol pro Mol Glucose eingesetzt und bei der Neutralisierung des sauren Katalysators mit Natriumhydroxid oder Natriumcarbonat ebenfalls pH-Werte zwischen 6,6 und 7 eingestellt.

Um die Alkylglykoside als Tensidrohstoffe in Wasch- und Reinigungsmitteln in dem dafür erforderlichen technischen Maßstab einsetzen zu können, müssen 2 Voraussetzungen erfüllt sein: Zum einen müssen die Alkylglykoside unter alkalischen Bedingungen farbstabil sein, damit sie in alkalischen Rezepturen verwendet werden können. Zum anderen aber müssen die Herstellungsverfahren für die Alkylglykoside so ausgelegt sein, daß diese Substanzen in großen Mengen problemlos hergestellt werden können. Beide Voraussetzungen aber werden durch die bekannten Herstellungsverfahren und die Eigenschaften der Verfahrensprodukte nicht erfüllt.

In der europäischen Patentanmeldung 77 167 (Rohm Haas, Arnaudis) wird zur Verbesserung der Farbqualität der oberflächenaktiven Alkylglykoside vorgeschlagen, daß bei deren Herstellung ein üblicher Säurekatalysator zusammen mit einem sauren Reduktionsmittel aus der Gruppe der phosphorigen Säure, hypophosphorigen Säure, schwefligen Säure, hyposchwefligen Säure, salpetrigen Säure und/oder hyposalpetrigen Säure bzw. mit deren Salzen, verwendet wird.

Nach der Lehre der europäischen Patentanmeldung 102 558 (BASF, Lorenz et al) werden hellfarbige C₃-C₅-Alkylglucoside, die zu oberflächenaktiven höheren Alkylglucosiden umacetalisiert werden können, durch Herstellung in Gegenwart eines sauren Katalysators und mindestens äquivalenten Mengen eines Alkalimetallsalzes einer Borsäure, vorzugsweise Natriumperborat, erhalten.

Nach einem weiteren Vorschlag der europäischen Patentanmeldung 165 721 (Staley, McDaniel et al) wird die wäßrige Lösung eines oberflächenaktiven Alkylpolyglucosids zunächst mit einem Oxidationsmittel, vorzugsweise mit einer Wasserstoffperoxidlösung und anschließend mit einer Schwefeldioxidquelle, z. B. mit einer wäßrigen Lösung von Natriumbisulfit, behandelt. Die so erhaltenen Produkte bleiben auch nach längerem Lagern farbstabil.

Allen bekannten Herstellungsverfahren, die sich mit der Verbesserung der Farbqualität und der Lagerstabilität von Alkylglykosiden befassen, haftet der Nachteil an, daß, um diese Verbesserungen zu erreichen, entweder zusätzliche chemische Wirkstoffe während des Herstellungsverfahrens zugesetzt werden müssen, oder aber ein solcher Zusatz für eine Nachbehandlung des Reaktionsprodukts erforderlich ist.

Ziel der vorliegenden Erfindung ist es, für die Herstellung von oberflächenaktiven Alkylglykosiden ein neues Verfahren bereitzustellen, bei dem die Verfahrensprodukte in einer solchen Qualität anfallen, daß eine Nachbehandlung zur Verbesserung des farblichen Aussehens und der Lagerstabilität nicht erforderlich ist. Ein weiteres Ziel der Erfindung ist es, die Verfahrensabläufe bei dem neuen Herstellungsverfahren so zu führen, daß man mit einem Minimum an chemischen Reaktionspartnern auskommt. Dabei sind außerdem die Verfahrensmerkmale so auszuwählen, daß eine Übertragung des Verfahrens in den großtechnischen Maßstab ohne Schwierigkeiten gelingt, und so die Herstellung von oberflächenaktiven Alkylglykosiden in solchen Mengen ermöglicht wird, daß diese Produkte als Tensid-Rohstoff von der Waschmittelindustrie in Wasch- und Reinigungsmitteln eingesetzt werden können.

Es wurde nun gefunden, daß sich diese und weitere Ziele durch eine neuartige Kombination von an sich bekannten sowie neuen Verfahrensmerkmalen zu einem Verfahren vom Typ des Umacetalisierungsverfahrens erreichen lassen.

Das erfindungsgemäße Verfahren zur Herstellung von oberflächenaktiven Alkylglykosiden mit im wesentlichen C₁₂- bis C₁₈-Alkyl- bzw. Alkenyl-Resten benutzt die Umacetalisierungsmethode mit Butanol und umfaßt die folgenden Verfahrensschritte:
a) Butanol wird zusammen mit einem sauren Katalysator im Reaktionsgefäß vorgelegt;
b) die Butanolmenge wird so gewählt, daß sie, bezogen auf 1 Mol der Glykose, 4 bis 10 Mol, vorzugsweise 6 bis 8 Mol, beträgt;
c) von der Butanolmenge wird ein Teil, vorzugsweise etwa die Hälfte des Butanols zusammen mit dem Katalysator vorgelegt und die andere Hälfte zur Suspendierung der Glykose verwendet;
d) als Katalysator wird eine sauer reagierende Verbindung, insbesondere eine Säure aus der Gruppe bestehend aus Schwefelsäure, Phosphorsäure, Paratoluolsulfonsäure und sulfosauren Ionenaustauscherharzen, in einer Menge von vorzugsweise 0,005 bis 0,02 Mol pro Mol der eingesetzten Glykose verwendet;
e) Erhitzen des Butanol/Katalysator-Gemisches auf Rückflußtemperatur;
f) Zugabe einer vorzugsweise vorgewärmten Suspension des übrigen Butanols und der Glykose unter portionsweiser oder kontinuierlicher Zudosierung unter Rühren so, daß das Reaktionsgemisch praktisch klar bleibt;
g) unmittelbares Abdestillieren des freiwerdenden Wassers als Butanol/Wasser-Gemisch;
h) es wird vorzugsweise ein leichtes Vakuum von etwa 800 bis 950 mbar während oder nach der Zudosierung der Butanol/Glykose-Mischung angelegt und unter weiterer Wärmezufuhr und Rühren die Abdestillation des Reaktionswassers beendet;
i) anschließend wird der vorgewärmte Fettalkohol in einer Menge von 2 bis 20 Mol pro Mol Glykose, vorzugsweise kontinuierlich zudosiert und gleichzeitig das Butanol im Vakuum abdestilliert;
j) die Abdestillation des Butanols wird so gesteuert, daß 0 bis 30 Mol-% Butylglykosid, bezogen auf 1 Mol der Glykose, im Reaktionsgemisch verbleiben;
k) das Reaktionsgemisch wird auf eine Temperatur unterhalb 95 °C abgekühlt und der saure Katalysator mit einer organischen oder anorganischen basischen Alkali-, Erdalkali- oder Aluminium- bzw. Alkali/Aluminiumverbindung neutralisiert und darüber hinaus auf einen pH-Wert von wenigstens 8, vorzugsweise etwa 9, eingestellt;
l) vorzugsweise wird eine Filtration des Reaktionsgemisches durchgeführt, und zwar vorzugsweise bei einer Temperatur von 80 bis 90 °C ;
m) der überschüssige Fettalkohol wird auf eine übliche, das Reaktionsprodukt schonende Weise auf einen Wert von unterhalb 5 Gew.-% abdestilliert.

Das so erhaltene Reaktionsprodukt wird vorzugsweise in an sich bekannter Weise durch Zusatz von Wasser zu einer leicht handhabbaren, ca. 60 %igen Paste verarbeitet. Das Reaktionsprodukt ist von gelblicher bis bräunlicher Färbung. Es wurde überraschenderweise gefunden, daß beim Lagern und insbesondere auch beim Weiterverarbeiten im alkalischen Milieu die ursprüngliche Farbqualität in ausreichender Weise erhalten bleibt. Für die meisten Anwendungszwecke des erfindungsgemäß hergestellten Alkylglykosids zur Herstellung von Wasch- und Reinigungsmitteln ist die direkt mit dem Verfahren erzielte Produktqualität völlig ausreichend; durch eine angeschlossene Bleichbehandlung mit Wasserstoffperoxid oder einer organischen Persäure kann die Farbqualität und Alkalistabilität noch verbessert werden.

Als Glykose wird bei dem erfindungsgemäßen Verfahren vorzugsweise Glucose verwendet. Üblicherweise liegt Glucose bekanntlich mit 1 Mol Kristallwasser vor. Diese kristallwasserhaltige Glucose kann ohne weiteres als Ausgangsmaterial bei dem erfindungsge mäßen Verfahren verwendet werden; allerdings ist es dann erforderlich, zusätzlich dieses Kristallwasser aus dem Reaktionsmilieu zu entfernen. Nachdem jedoch wasserfreie Glucose in großen Mengen als Ausgangsmaterial zugänglich ist, wird besonders bevorzugt wasserfreie Glucose als feinteiliges Pulver verwendet. Als saurer Katalysator wird vorzugsweise wegen ihrer im Vergleich zu Schwefelsäure geringeren korrodierenden Wirkung gegenüber Geräten und Leitungen aus Stahl die Paratoluolsulfonsäure verwendet. Prinzipiell sind aber als Katalysatoren alle sauren Verbindungen, einschließlich der sogenannten Lewis-Säuren, welche die Acetalisierungsreaktion zwischen Fettalkohol und Zuckermolekül katalysieren, geeignet.

Für eine sofortige, d. h. unmittelbar nach der Freisetzung erfolgende Abdestillation des Reaktionswassers, ist die Einstellung eines Temperaturgleichgewichts im Reaktionsgefäß erforderlich. Dazu wird vor der Umsetzung mit der Glykose das im Reaktionsgefäß vorgelegte Butanol auf Rückflußtemperatur gebracht und die Destillationskolonne bei unendlichem Rücklauf betrieben. Im Falle des erfindungsgemäß verwendeten n-Butanols beträgt die Rückflußtemperatur 118 °C; mit der Bildung des niedriger siedenden Butanol/Wasser-Gemisches stellt sich eine Brüdentemperatur von 95 bis 110 °C ein. Nach Phasentrennung des Butanol/Wasser-Destillats kann die butanolreiche Phase wieder in den Kolonnenraum zurückgeführt werden, obwohl sie gelöstes Wasser enthält. Bei der Durchführung des Verfahrens im Labormaßstab ist es allerdings einfacher, die abdestillierte Butanolmenge wieder durch frisches Butanol zu ersetzen. Bei der Durchführung des Verfahrens im großtechnischen Maßstab kann die wasserenthaltende Butanolphase ohne weiteres wieder in die Kolonne zurückgeführt werden; dort bleibt das Wasser der Butanolphase quasi stationär und kommt nicht mehr mit dem Reaktionsgemisch in Kontakt. Ein leichtes Vakuum von etwa 800 bis 950 mbar kann auch bereits während des Aufheizens des Butanols angewendet werden, um so die Rückflußtemperatur auf die Höhe der Butanol/Wasser-Brüdentemperatur einzustellen.

Beim Zuführen der Wärmeenergie, die zur Entfernung des Butanol/Wasser-Gemisches und zur Aufrechterhaltung der Reaktionstemperatur benötigt wird, ist es wesentlich, daß zwischen Reaktorwand und Reaktionsgemisch nur eine geringe Temperaturdifferenz vorhanden ist, damit Überhitzungen vermieden werden. Um diese geringe Temperaturdifferenz einzustellen, genügt es bei Ansätzen im Labor, ein übliches Öl-Bad mit Thermostat zu verwenden und gleichzeitig das Reaktionsgemisch kräftig zu rühren. Bei Ansätzen im technischen Maßstab hat sich als besonders vorteilhaft erwiesen, die Wärmeenergie über einen externen Kreislauf, vorzugsweise bestehend aus einer Pumpe und einem Wärmeaustauscher, vorzunehmen. Zu diesem Zweck wird ständig ein Teil des Reaktionsgemisches über eine Rohrleitung abgezogen, im Wärmeaustauscher erwärmt und wieder in den Reaktor zurückgeführt. Auf diese Weise ist es möglich, hohe Reaktorwandtemperaturen, d. h. solche von mehr als 125 °C zu vermeiden und damit eine negative Auswirkung der Temperaturführung auf die Farbwerte des Endproduktes zu verhindern.

Die Suspension der Glykose in Butanol, vorzugsweise wird wasserfreie Glucose verwendet, wird nach einer bevorzugten Ausführungsform des Verfahrens einer Vorbehandlung im Sinne einer Feindispergierung unterworfen. Für Laboransätze haben sich dafür die Verwendung eines hochtourigen üblichen Laborrührers oder aber eine Ultraschallbehandlung als geeignet erwiesen. Bei großtechnischen Ansätzen wird zur feinen Dispergierung mit Vorteil ein Inline-Mischer, beispielsweise ein Stator/Rotor-Mischer verwendet. Diese Feindispergierungsmaßnahme hat den erwünschten Nebeneffekt, daß sich dabei die Suspension erwärmt. Die Zugabe der Glykose-Suspension in das Reaktionsgefäß erfolgt entweder portionsweise oder kontinuierlich, wobei man im Falle von Laboransätzen der portionsweisen Zugabe und im Falle von großtechnischen Ansätzen der kontinuierlichen Zugabe den Vorteil gibt. In beiden Fällen sind die Dosierungsraten bzw. die Zeitintervalle zwischen den Dosierportionen so zu wählen, daß das Reaktionsgemisch praktisch klar bleibt, d. h. eine homogene Phase bildet. Dieser Begriff ist hier so zu verstehen, daß bei portionsweiser Zugabe zunächst eine kurze Periode auftritt, in der das Reaktionsgemisch getrübt ist, daß diese Trübung jedoch als Folge der Veretherungsreaktion bzw. Lösung wieder verschwindet. Zweckmäßigerweise wird erst danach die nächste Glykoseportion hinzugegeben. Bei der Variante der kontinuierlichen Zugabe der Glykose tritt während der gesamten Zugabezeit eine geringe Trübung des Reaktionsgemisches auf, da ständig geringe Mengen von nichtumgesetzter Glykose vorliegen. Hier ist darauf zu achten, daß die Zugaberate so gesteuert wird, daß der Grad der Trübung gleichmäßig bleibt und jedenfalls sich nicht verstärkt, bzw. daß die Trübung beim Abbrechen der Zudosierung rasch unter Bilden einer klaren Phase verschwindet.

Vorzugsweise wird die Acetalisierung mit dem Butanol in einem leichten Vakuum, d. h. bei einem Druck von etwa 800 - 950 mbar durchgeführt.

Anstelle des n-Butanols können im Prinzip für die Acetalisierung auch andere kurzkettige Alkanole oder Alkandiole ähnlicher Molekülgröße wie z. B. Propanol, Pentanol, Hexanol oder Propylenglykol verwendet werden; doch ist n-Butanol wegen der Summe seiner vorteilhaften Eigenschaften wie z. B. Siedepunkt, Abtrennbarkeit von den Fettalkoholen, Vermischbarkeit des Butylglykosids mit den Fettalkoholen die bevorzugte Substanz.

Nach dem Ausreagieren der Glykose, was man an dem Aufhören der Bildung von Reaktionswasser erkennen kann, wird der vorzugsweise auf Reaktionstemperatur vorgewärmte Fettalkohol in der gewählten Menge, die vorzugsweise 5 bis 7 Mol pro Mol Glykose beträgt, so unter Vakuum in das Reaktionsgefäß hinzudosiert, daß man gleichzeitig das freiwerdende Butanol abdestillieren kann.

Als primäre Alkohole werden insbesondere die Fettalkohole, d. h. die höheren aliphatischen primären C₁₂- bis C₁₈-Alkohole verwendet, wobei es sich vorzugsweise um gesättigte und insbesondere um geradkettige Alkohole, wie sie durch die Hydrierung von nativen Fettsäuren im technischen Maßstab erhalten werden können, handelt. Typische Vertreter der höheren aliphatischen Alkohole, die nach dem erfindungsgemäßen Verfahren verwendet werden können, sind z. B. die Verbindungen n-Dodecylalkohol, n-Tetradecylalkohol, n-Hexadecylalkohol, n-Octadecylalkohol, n-Octylalkohol, n-Decylalkohol, Undecylalkohol, Tridecylalkohol. Da die Fettalkohole bevorzugt aus natürlichen Fettquellen stammen, kommen üblicherweise auch Gemische technischer Fettalkohole als Reaktionspartner in Betracht, beispielsweise ein technisches Gemisch aus etwa 3 Gewichtsteilen Laurylalkohol und 1 Gewichtsteil Myristylalkohol. Zwar ist im Prinzip jeder längerkettige Alkohol, der eine primäre Alkoholgruppe enthält, für die Umsetzung geeignet, also auch primäre Alkohole mit Anteilen an verzweigten Kohlenstoffketten, wie z. B. die sogenannten Oxoalkohole; jedoch liegt der Schwerpunkt des Verfahrens bei der Herstellung von Tensiden, die ausschließlich aus nachwachsenden Rohstoffen herstellbar sind.

Als Vakuum werden hier Unterdrücke bis herab zu 10 mbar eingestellt. Durch diesen synchronen Austausch von Fettalkohol gegen Butanol erzielt man nicht nur eine erwünschte hohe Raum/Zeit-Ausbeute, weil man so die Reaktionsgefäße relativ klein dimensionieren kann, sondern es hat sich gezeigt, daß man auf diese Weise auch den Anteil an Alkylmonoglykosiden im Reaktionsprodukt in Richtung des Entstehens höherer Anteile beeinflussen kann. Nach einer bevorzugten Variante wird dieses Verfahrensmerkmal der Fettalkoholzugabe so gestaltet, daß man unterge ordnete Mengen des Fettalkohols, worunter 0,5 bis 10 Gew.-% des gesamten Fettalkohols der zum Einsatz gelangt, verstanden werden, bereits mit der feindispergierten Butanol/Glykose-Suspension in die Reaktion einführt. Auf diese Weise läßt sich die Stabilisierung der Glykose/Butanol-Dispersion verbessern. Nach Beendigung der Fettalkohol-Zudosierung wird im allgemeinen noch nachgerührt, um auf diese Weise die Umacetalisierungsreaktion bis zu dem jeweils gewünschten Umsetzungsgrad zu steuern. Dabei ist es nach diesem Verfahren prinzipiell möglich, die Umacetalisierungsreaktion so zu führen, daß praktisch kein Butylglykosid mehr im Reaktionsgemisch vorhanden ist, worunter man versteht, daß die Butylglykosidanteile unterhalb von 1 Gew.-% liegen. In vielen Fällen ist es jedoch erwünscht, daß das Endprodukt noch bestimmte Anteile an Butylglykosid enthält.

Es hat sich gezeigt, daß die Anwesenheit von Butylglykosid im Reaktionsendprodukt in mehrfacher Hinsicht von Vorteil ist, so daß man nach einer bevorzugten Ausführungsform das Verfahren so lenkt, daß wenigstens 2 und maximal 30 Gew.-% Butylglykosid im Reaktionsgemisch verbleiben. Durch den Butylglykosid-Anteil wird die Fließfähigkeit des Reaktionsgemisches verbessert, so daß sich der Fettalkohol-Überschuß leichter abdestillieren läßt. Auch die Hellfarbigkeit und Alkalistabilität des Endprodukts wird durch die Anwesenheit des Butylglykosids vorteilhaft beeinflußt; schließlich macht sich der Butylglykosidanteil auch anwendungstechnisch vorteilhaft bemerkbar.

Um die gewünschten Butylglykosidanteile zu erhalten, wird nach dem Abdestillieren des Butanols und vor der Neutralisation des Katalysators vorzugsweise noch eine Nachrührzeit von bis zu ca. 1 Stunde vorgesehen, während der man das Reaktionsgemisch bei Normaldruck und einer Temperatur zwischen 100 und 115 °, insbesondere bei ca. 110 °, rührt. Auf diese Weise kann die Umsetzung des Butylglykosids mit dem Fettalkohol gezielt weitergeführt werden. Der Restgehalt an Butylglykosid kann durch Bestimmung der abdestillierten Butanolmenge oder durch Analysen von Produktproben ermittelt werden.

Für die Neutralisation des Katalysators eignen sich prinzipiell alkalisch reagierende organische oder anorganische Verbindungen, deren Neutralisationsprodukte die weitere Aufarbeitung bzw. Anwendung nicht beeinträchtigen. Vorzugsweise werden solche alkalischen Verbindungen benutzt, deren Neutralisationsprodukte die Filtrierbarkeit des schwachalkalisch (wenigstens pH 8) eingestellen Reaktionsgemisches nicht beeinträchtigen bzw. verbessern. Besonders bevorzugt sind solche alkalischen Verbindungen, die bei der Neutralisation kein freies Neutralisationswasser bilden. Geeignete anorganische und organische alkalische Verbindungen sind beispielsweise Calciumhydroxid, Magnesiumhydroxid, Magnesiumoxid oder -carbonat, Natriumcarbonat, Kaliumcarbonat, Natriummethylat oder -ethylat, Magnesiummethylat oder -ethylat, Natrium- bzw. Magnesiumpropylat oder -butylat, vorzugsweise die Magnesiumalkoholate oder Magnesiumoxid, sowie die Zeolithe NaA und NaX, bzw. NaA in Mischung mit Calciumhydroxid (Verhältnis 10 : 1 bis 1 : 1), wobei der Zeolith NaA vorzugsweise weniger gebundenes Wasser als dem Gleichgewichtswert entspricht, enthält.

Nach der Zugabe der alkalischen Verbindung unter Rühren und Einstellen des leicht alkalischen pH-Wertes von wenigstens pH 8, vorzugsweise pH 9 bis 10, wird vorzugsweise das Reaktionsgemisch bei ca. 80 bis 100 °C filtriert, wobei für Laboransätze die üblichen Filternutschen verwendet werden. Für die Filtration technischer Ansätze werden im allgemeinen Gewebefilter benutzt. Nach einer weniger bevorzugten Ausführungsform des Verfahrens wird das Reaktionsgemisch nach der Stufe der Neutralisation und des Alkalischmachens nicht filtriert. In diesem Fall sind zweckmäßigerweise sowohl der saure Katalysator als auch die alkalische Verbindung zum Desaktivieren des Katalysators so zu wählen, daß ihre Umsetzungsprodukte im Reaktionsgemisch dessen Anwendung nicht beeinträchtigen. Beispielsweise kommen dann saure Ionenaustauscherharze als Katalysatoren und basische Calciumverbindungen bzw. Silikate weniger bzw. nicht in Betracht.

Für die Abdestillation des Fettalkoholüberschusses sind produktschonende Vakuumdestillations-Methoden zu wählen, bei denen die sogenannte Sumpftemperatur bei solchen Werten gehalten werden kann, bei denen das Alkylglykosid thermisch stabil ist. Dies bedeutet, daß die Sumpftemperatur den Wert von 160 °C möglichst nicht überschreiten soll. Für Destillationen von Laboransätzen können die dafür üblichen Vakuumsdestillationsgeräte bei einem Vakuum von ca. 0,01 mbar benutzt werden. Bei technischen Ansätzen im Produktionsmaßstab wird die Abdestillation des Fettalkohols vorzugsweise nach einem zweistufigen Verfahren durchgeführt, wobei in einer ersten Stufe eine Abreicherung des Fettalkoholanteils auf Werte von ca. 40 bis ca. 20 % mit einem Dünnschichtverdampfer oder einem Fallfilmverdampfer durchgeführt wird. Diese erste Stufe dient auch der Entgasung des Reaktionsgemisches. In einer zweiten Stufe wird mit einem Kurzwegverdampfer die weitere Fettalkohol-Abreicherung auf den gewünschten Endwert eingestellt. Dieser Endwert kann, bezogen auf das Endprodukt, bei Werten unterhalb 0,5 Gew.-% liegen, wenn das Produkt praktisch frei von Fettalkohol sein soll. Für den Fall, daß gezielt Fettalkoholanteile im Endprodukt erwünscht sind, können die Werte bei 3 bis 5 Gew.-% Fettalkohol liegen. Beispielsweise hat sich gezeigt, daß Verfahrensendprodukte mit einem Fettalkoholanteil von über 2 Gew.-%, vorzugsweise von 3 bis 5 Gew.-% anwendungstechnische Vorteile bringen.

Für die schonende Auftrennung von temperaturempfindlichen Substanzgemischen gilt generell, daß sich zur schonenden Verdampfung bei reduziertem Druck Fallfilmverdampfer und insbesondere Dünnschichtverdampfer besonders gut eignen, weil sich in diesen Geräten extrem kurze Verweilzeiten bei den erforderlichen höheren Temperaturen erreichen lassen. In dem vorliegenden Fall eignet sich zur Abreicherung des überschüssigen Fettalkohols mit 10 bis 18 Kohlenstoffatomen vom Alkylglykosid als eigentlichem Produkt besonders der Dünnschichtverdampfer. Als Dünnschichtverdampfer bezeichnet man solche Verdampfer, in denen ein hochviskoses schwer siedendes Gemisch auf eine beheizte Wand aufgegeben und dort durch rotierende Wischelemente mechanisch verteilt wird. Dabei werden dünne Flüssigkeitsschichten bzw. Flüssigkeitsfilme erzeugt, und die Filmoberflächen werden ständig erneuert. Die entstehenden Dämpfe strömen entgegen dem Fluß des Produktfilms und verlassen den Verdampfer in den außen angeordneten Kondensator. Im Dünnschichtverdampfer wird im allgemeinen bei Drucken von nur einigen mbar gearbeitet und die Verweildauer für das Produkt beträgt nur wenige Sekunden.

In einer 2-stufigen Anlage, wie sie in dem erfindungsgemäßen Verfahren bevorzugt benutzt wird, fungiert der Dünnschichtverdampfer auch als Vorentgaserstufe für den in zweiter Stufe benutzten Kurzwegverdampfer. Permanente Gase, die in der viskosen Flüssigkeit gelöst sind, werden so im Zuge der Abreicherung des Reaktionsproduktes an überschüssigem Fettalkohol im Dünnschichtverdampfer aus der Flüssigkeit entfernt.

Bei den bevorzugt eingesetzten Kurzwegverdampfern handelt es sich im Prinzip um Wischfilmverdampfer mit einem im Verdampfer eingebauten Kondensator. Diese Geräte eignen sich zur Destillation hochsiedender und temperaturempfindlicher Produkte im Bereich 10⁻¹ bis 10⁻⁴ mbar. Ähnlich wie bei dem Dünnschichtverdampfer wird auch bei dem Kurzwegverdampfer die Flüssigkeit mechanisch auf der Heizfläche durch Wischer verteilt. Erfindungsgemäß wird im Kurzwegverdampfer als zweiter Stufe der überschüssige Alkohol auf praktisch beliebige Restgehalte, die unter 1 % liegen können, entfernt. Die 2-Stufen-Anordnung mit Dünnschichtverdampfer und Kurzwegverdampfer gestattet hohe Durchsätze in Verbindung mit der gezielten Einstellung des erwünschten Restgehaltes an Fettalkohol im Endprodukt. Für technische Zwecke lassen sich Dünnschicht- und Kurzwegverdampfer so dimensionieren, daß Durchsätze von bis zu 300 kg/qm und Stunde, bezogen auf den Dünnschichtverdampfer, ohne weiteres möglich sind. Die erfindungsgemäß bevorzugte Verfahrensvariante mit der 2-stufigen Fettalkoholabreicherungsanlage läßt sich prinzipiell auch in der passenden Dimensionierung für die Aufarbeitung von Laboransätzen verwenden.

Im Anschluß an das eigentliche Alkylglykosid-Herstellungsverfahren und nach Entfernung des Fettalkohol-Überschusses wird das Reaktions-Endprodukt, das im erkalteten Zustand eine schwach gelbliche wachsartige Masse bildet, vorzugsweise wegen der besseren Handhabbarkeit in eine wäßrige Paste mit ca. 60 % Wirkstoffgehalt übergeführt. In besonderen Fällen, wenn hohe Ansprüche an die Farblosigkeit des Endprodukts gestellt werden, kann gleichzeitig mit der Herstellung der wäßrigen Paste eine Bleiche mit Wasserstoffperoxid oder einer organischen Persäure, wie z. B. Dodecandipersäure vorgenommen werden. Bei korrekter Durchführung des erfindungsgemäßen Verfahrens ist jedoch im allgemeinen eine Bleichung des Endprodukts nicht erforderlich.

Alkylglykosid-Gemische, wie sie nach dem hier beschriebenen und beanspruchten Verfahren erhalten werden können, enthalten eine Menge an Alkylmonoglykosid, die bezogen auf die Gesamtmenge an Alkylmonoglykosid und Alkyloligoglykosid, deutlich über 70 Gew.-%, vorzugsweise bei 70 bis 90 Gew.-%, und insbesondere bei 75 bis 90 Gew.-% liegt. Dabei setzt sich die Gesamtmenge aus Alkylmonoglykosid und Alkyl oligoglykosid so zusammen, daß rein rechnerisch der mittlere Oligomerisierungsgrad maximal 1,35 beträgt. Als weitere wesentliche Komponente enthält das Alkylglykosid Polyglykosen, d. h. das Verfahrensproduct besteht in erster Linie im wesentlichen aus den drei Komponenten Alkylmonoglykosid, Alkyloligoglykosid und Polyglykose.

Dies bedeutet im vorliegenden Fall, daß einerseits der Anteil an kurzkettigen Alkylglykosiden, beispielsweise Butylglykosid, durch die angepaßte Verfahrensführung so reduziert worden ist, daß im Verfahrensprodukt weniger als 1 Gew.-% vorliegt und daß die Abdestillation des überschüssigen Fettalkohols so weit durchgeführt worden ist, daß davon weniger als 0,5 Gew.-% im Produkt verbleiben. Unter Alkyloligoglykosiden werden hier die Alkyldiglykoside, die Alkyltriglykoside und höhere Homologe verstanden, wie sie nach gängigen Analysenmethoden noch eindeutig erfaßt und zugeordnet werden können. Bei den nach dem erfindungsgemäßen Verfahren hergestellten Produkten bestanden diese Alkyloligoglykoside praktisch nur aus den Di- und Triglykosidverbindungen. Bei der dritten Komponente handelt es sich um Polyglykosen, die bei der Alkylierungsreaktion in einer Nebenreaktion durch Kondensation der Glykosemoleküle untereinander gebildet werden. Das durchschnittliche Molekulargewicht dieser Polyglykosen liegt bei 2000 bis 10 000. Es wurde überraschenderweise gefunden, daß die Anwesenheit dieser Polyglykosen die Lager- und Alkalibeständigkeit das Produkts nicht beeinträchtigt, und daß darüber hinaus die Tensidwirkung des Alkylglykosids, d. h. des Gemisches aus Alkylmonoglykosid und Alkyloligoglykosid nicht vermindert wird.

Vorzugsweise besteht das Alkylglykosid-Gemisch im wesentlichen aus der Dreier-Kombination aus 50 bis 95 Gew.-%, vorzugsweise 65 bis 91 Gew.-% Alkylmonoglykosid, 2 bis 25 Gew.-%, vorzugs weise 4 bis 20 Gew.-% Alkyloligoglykosid, und 5 bis 30 Gew.-%, vorzugsweise 5 bis 25 Gew.-% Polyglykose.

Das erfindungsgemäße Verfahren kann auch eine Viererkombination ergeben, bestehend im wesentlichen aus: 45 bis 90 Gew.-%, vorzugsweise 50 bis 90 Gew.-% Alkylmonoglykosid, 2 bis 22 Gew.-%, vorzugsweise 3 bis 20 Gew.-% Alkyloligoglykosid, 4 - 25 Gew.-% Polyglykose und 3 bis 30 Gew.-% Butylglykosid.

Der Ausdruck "bestehend im wesentlichen aus" soll auch hier bedeuten, daß Anteile an überschüssigem Fettalkohol praktisch vollständig, d. h. auf Werte von weniger als 0,5 Gew.-% destillativ entfernt worden sind. Auch hier setzt sich die Gesamtmenge aus Alkylmono- und Alkyloligoglykosid so zusammen, daß rein rechnerisch der mittlere Oligomerisierungsrad maximal 1.35 beträgt. Die bevorzugte Glykose des Verfahrens ist die Glucose; es können aber in untergeordneten Mengen auch Isomerisierungsprodukte, die nicht zu den wesentlichen Bestandteilen zählen, vorliegen.

Auch eine Fünfer-Kombination, die zusätzlich als 5. Komponente noch 0,5 - 5, vorzugsweise 2,5 - 4 Gew.-% Fettalkohol enthält, wobei die übrigen vier wesentlichen Bestandteile in einer entsprechend verminderten Menge vorliegen, kann als Verfahrensprodukt anfallen. Ein wesentlicher Anteil an freiem Fettalkohol mit 12 - 18 Kohlenstoffatomen kann in vielen Fällen aus anwendungstechnischen Gründen erwünscht sein. Dieser Fettalkoholanteil wird am einfachsten dadurch eingestellt, daß man die destillative Abreicherung des überschüssigen Fettalkohols nur bis zu der gewünschten Endmenge durchführt.

Für die Verfahrensprodukte wurde die Menge des überschüssigen Fettalkohols gaschromatographisch bestimmt. Die Bestimmung der Alkylmono- und Alkyloligoglucosid-Gehalte wurde entweder mittels Hochleistungsflüssigchromatographie (HPLC) oder mittels Gaschromatographie (GC) durchgeführt. Die Peak-Zuordnung bei der GC-Methode erfolgte durch Kopplung mit dem Massenspektrometer und Vergleich mit Standardsubstanzen. Die HPLC-Peakzuordnung wurde durch Fraktionierung und NMR-spektroskopische Identifizierung der Fraktionen sowie durch Vergleich mit Standards vorgenommen. Die Anteile an Polyglucose wurden mittels präparativer HPLC isoliert. Die analytische Identifizierung der auf diese Weise isolierten Komponenten erfolgte NMR-spektroskopisch sowie über enzymatische Zuckertests. Der molekulare Gewichtsbereich der Polyglucose wurde durch Gelpermeationschromatographie (GPC) bestimmt. Nebenprodukte, die beispielsweise durch Isomerisierung der Glucose zu Fructose oder durch Reaktion der Fettalkohole mit sich selber entstanden sein konnten, bzw. die durch die Neutralisation des sauren Katalysators gebildeten Salze, wurden nicht näher identifiziert.

### Beispiele

Die folgenden Beispiele wurden alle mit Glucose als der bevorzugten Glykose durchgeführt. In den meisten Beispielen wurde die Glucose in wasserfreier handelsüblicher Form verwendet. Prinzipiell kann jedoch auch die 1 Mol Wasser enthaltende übliche Dextrose eingesetzt werden.

### Beispiel 1: Dieses Beispiel beschreibt das erfindungsgemäße Verfahren unter Verwendung von wasserfreier Glucose im Labormaßstab.

Normal-Butanol wurde in einer Menge von 222g (3 Mol) in einem 2 - Liter Mehrhalskolben mit Rührer, Thermometer und Tropftrichter und Destillationsaufsatz zur Wasserabscheidung vorgelegt und dazu 2,2g (11,2 mMol) Paratoluolsulfonsäure als Katalysator gegeben. Die Mischung wurde auf 110 °C erhitzt. Dann wurde eine Suspension von 180 g (1 Mol) wasserfreier Glucose in weiteren 222 g (3 Mol) Normal-Butanol portionsweise, und zwar in 10 Portionen, in Abständen von 5 Minuten hinzu dosiert. Dabei bildete sich ein klares Reaktionsgemisch. Während des Dosierungsvorganges wurde die Hauptmenge des entstehenden Reaktionswassers zusammen mit Butanol bei Normaldruck abdestilliert (Destillatmenge: 90,7 g, Wassergehalt 14,3 % bestimmt nach Karl Fischer) Anschließend wurden 1164 g (6 Mol) eines auf circa 80° C vorgewärmten C₁₂/C₁₄ - Fettalkohols (Gemisch aus circa 75 Gew.-% Dodecanol und circa 25 Gew.-% Tetradecanol) während 75 Minuten kontinuierlich zum Reaktionsgemisch gegeben, wobei gleichzeitig weiter Butanol abdestilliert wurde. Dazu wurde zunächst der Druck auf 800 mbar eingestellt und anschließend allmählich auf 10 mbar reduziert. (Destillatmenge 334 g). Nach dem Abdestillieren des Butanols wurde das Gemisch noch 30 Minuten lang bei 110 °C und Normaldruck gerührt und dann auf 90 °C abgekühlt. Anschließend wurde durch Zusatz von 1,93 g (16,9 mMol) Magnesiumethylat der Katalysator desaktiviert, wozu man weitere 30 Minuten bei 90 °C rührte. Das Reaktionsgemisch hatte anschließend einen pH-Wert von 9-10. Nach Filtration bei 90 °C durch eine beheizte Nutsche wurde das Produkt zur Abtrennung des überschüssigen Fettalkohols im Vakuum bei 0,01 mbar und einer Sumpftemperatur von maximal 160°C destilliert. Die Destillatmenge betrug 1044 g, der Destillationsrückstand, also das Endprodukt, betrug 298 g. Dieser Rückstand wurde bei 70 bis 80 °C mit Wasser zu einer 60-%igen Paste verarbeitet.

Produktkenndaten: OH-Zahl 691; Säurezahl 2,0; restlicher Fettalkohol 3,1 Gew.-%; Butylglucosid 18,0 Gew.-% Dodecyl/Tetradecylmonoglucosid 54 Gew.-%; Dodecyl/Tetradecyloligoglucosid (hauptsächlich Maltosid) 3,5 Gew.-%; Polyglucose circa 20 gew.-% (MG ca. 2500). Diese Werte wurden mit HPLC-Methoden bestimmt.
Farbwerte des Produktes (40 %ig in Wasser/Isopropylalkohol):
Lovibond-Farbzahlen 6 (gelb); 1,4 (rot). Eine Probe der wäßrigen Paste wurde mit 0,5 % H₂0₂ - (bezogen auf das Produkt) als 35 %ige Lösung versetzt. Dann wurde 1 Stunde lang bei 80 °C gerührt. Ein pH-Wert von ca. 8 wurde durch Zugabe von NaOH eingestellt. Lovibondzahlen nach der Bleiche: 0,8 (gelb); 0,3 (rot). Ein ähnliches Produkt wird erhalten, wenn man anstelle des C₁₂/C₁₄-Fettalkohols äquivalente Mengen eines C₁₂/C₁₃-Oxoalkohols mit ca. 25 % hauptsächlich 2-Methylverzweigung (Dobanol 23) einsetzt.

### Beispiel 2: Dieses Beispiel beschreibt das Verfahren unter Verwendung von wasserhaltiger Glucose.

Ansatz und Verfahrensdurchführung wie in Beispiel 1; die Glu
cose wurde jedoch als 198 g Dextrose (Glucose mit 1 Mol Wasser) eingesetzt. Außer daß bei der Acetalisierung mit Butanol die zu sätzliche Wassermenge von circa 18 g abdestilliert wurde, waren keine wesentlichen Abweichungen zum Beispiel 1 festzustellen.

### Beispiel 3: Dieses Beispiel beschreibt den Lager- und Alkalistabilitätstest des erfindungsgemäß hergestellten Produkts.

Eine 60 %ige wäßrige Paste des Produkts wurde mit konz. NaOH auf pH 12 - 13 eingestellt und 0,5 Stunden lang auf 100 °C erhitzt. Nach dem Abkühlen auf Raumtemperatur wurde durch Zugabe von Isopropylalkohol auf 40 Gew.-% Aktivsubstanzgehalt (Produktmenge) eingestellt. Dann wurden die Farbzahlen (rot und gelb) nach Lovibond gemessen. Es wurde eine 1-Zoll-Küvette verwendet (siehe DGF-Einheitsmethoden, Abteilung C, Fette, C-IV 46 (52), Wissenschaftliche Verlagsgesellschaft mbH, Stuttgart, 1984).

Das Produkt des Beispiels 1 wurde nach dieser Vorschrift behandelt. Anschließend wurden die Lovibond-Farbzahlen mit 18 (gelb) und 3 (rot) ermittelt.
Anwendungstechnische Versuche mit dem Produkt des Beispiels 1 haben gezeigt, daß für die meisten Verwendungszwecke das erfindungsgemäße Produkt mit dieser Farbqualität als Eigenschaft nach dem obigen Testverfahren ausreicht.

Mit dem gebleichten Produkt des Beispiels 1 wurde ebenfalls der Alkalistabilitätstest durchgeführt. Danach wurden die unveränderten Lovibond-Farbzahlen 0,8 (gelb) und 0,3 (rot) gemessen. Das besonders alkalistabile gebleichte Produkt empfiehlt sich für solche Einsatzzwecke, wo hohe Anforderungen an den Weißgrad einer Formulierung gestellt werden.

### Beispiel 4: Dieses Beispiel beschreibt die Durchführung des Verfahrens im Produktionsmaßstab.

Es kamen die folgenden Stoffmengen zum Einsatz:
Dodecyl/Tetradecylalkohol-Mischung (Lorol S) 1620 kg;
Butanol 871 kg;
Paratoluolsulfonsäure 3,5 kg;
Magnesiumethylat 2,0 kg;
Glucose (wasserfrei) (Puridex feinkörnig) 250 kg.

In einer Reaktionseinheit bestehend aus einem 3,2 m³ Reaktor mit Destillationskolonne und einem externen Flüssigkeitskreislauf aus einer Pumpe und einem Wärmetauscher wurde die Hälfte der Butanolmenge zusammen mit dem Katalysator vorgelegt und auf 114 °C erhitzt. Dann wurde eine Suspension der Glucose in der restlichen Butanolmenge zunächst in einem Stator/Rotor-Mischer (Typ Supraton) feindispergiert, wobei sich diese Suspension auf 75°C erwärmte. Die Zugabe der Glucose/Butanol-Suspension erfolgte kontinuierlich innerhalb von 1,3 Stunden. Während dieser Zeit wurden 65 kg eines Butanol/Wasser-Gemisches bei einem Unterdruck von 900 mbar abdestilliert. Die zur Entfernung dieses Gemisches und zur Aufrechterhaltung der Reaktionstemperatur notwendige Energie wurde über einen externen Flüssigkeitskreislauf, bestehend aus einer Pumpe und einem Wärmetauscher, durch den das Reaktionsgemisch geleitet wurde, gewährleistet. Die nach der Phasentrennung des Butanol/Wasser-Gemisches erhaltene wassergesättigte Butanolphase wurde auf den Kopf der Kolonne zurückgeführt. Nach der Bildung des Butylglucosids befanden sich 725 kg freies Butanol im Reaktionsgemisch. Im Anschluß an die Butylglucosid-Bildung wurde bei einem Unterdruck von 800 bis 10 mbar der auf Reaktionstemperatur vorgewärmte Fettalkohol kontinuierlich hinzugegeben und gleichzeitig das frei werdende Butanol abgetrennt. Für die Austauschreaktion Butanol/Fettalkohol und für die Abdestillation des Restbutanols wurden insgesamt 1,8 Stunden Reaktionszeit benötigt. Nach einer Nachreaktionszeit von 1 Stunde bei 110 °C und einem Druck von 1013 mbar wurde auf 90 °C abgekühlt und dann das Magnesiumethylat zur Desaktivierung des Katalysators hinzu gegeben. Nach 30 Minuten wurde ein pH-Wert des Reaktionsgemisches von 8,5 gemessen. Nach der Filtration bei 85 °C durch einen Beutelfilter wurden 1885kg des Reaktionsgemisches in einen Dünnschichtverdampfer Typ Sambay (0,75 qm Verdampferfläche, 8 mbar, ca. 170 °C) geleitet und der überschüssige Fettalkohol bis auf einen Abreicherungswert von ca. 32 % abgetrennt. Das bei 135 °C gehaltene Produkt war niedrig viskos und konnte leicht in einen Kurzwegverdampfer mit Rollenwischer vom Typ KD 75, Fa. Leybold, übergeführt werden. Der Kurzwegverdampfer wurde unter den folgenden Bedingungen betrieben: Verdampferfläche 0,75 qm; Arbeitsdruck 0,075 mbar, im Verdampfer gemessen; Beheizungstemperatur 160 °C; Sumpfablauftemperatur 152 °C.
Das Produkt wog nach Verlassen des Kurzwegverdampfers 436 kg; Fettalkoholgehalt 4,9 Gew.-%. Lovibond-Farbwerte (des 40 %igen Produkts): 5,5 (gelb) und 1,3 (rot).
Zusammensetzung des Produkts (mit GC und HPLC ermittelt): Monoglucosid 55 Gew.-%, Oligoglucoside 18 Gew.-%, Oligomerisierungsgrad 1,3 (berechnet aufgrund der Gewichtsanteile), Polyglucose 8 Gew.-%, Butylglucoside 12 Gew.-%., Fettalkohol 3 Gew.-%.

## Patentansprüche

1. Verfahren zur Herstellung von oberflächenaktiven Alkylglykosiden mit im wesentlichen C₁₂- bis C₁₈-Alkyl- bzw. Alkenylresten nach der Umacetalisierungsmethode mit Butanol, umfassend die folgenden Verfahrensschritte:
a) Butanol wird zusammen mit einem sauren Katalysator im Reaktionsgefäß vorgelegt;
b) die Butanolmenge wird so gewählt, daß sie, bezogen auf 1 Mol der Glykose, 4 bis 10 Mol, vorzugsweise 6 bis 8 Mol, beträgt;
c) von der Butanolmenge wird ein Teil, vorzugsweise etwa die Hälfte zusammen mit dem Katalysator vorgelegt und die andere Hälfte zur Suspendierung der Glykose verwendet:
d) als Katalysator wird eine sauer reagierende Verbindung, insbesondere eine Säure aus der Gruppe bestehend aus Schwefelsäure, Phosphorsäure, Paratoluolsulfonsäure und sulfosauren lonenaustauscherharzen, in einer Menge von vorzugsweise 0,005 bis 0,02 Mol pro Mol der eingesetzten Glykose verwendet;
e) Erhitzen des Butanol/Katalysator-Gemisches auf Rückflußtemperatur;
f) Zugabe einer vorzugsweise vorgewärmten Suspension des übrigen Butanols und der Glykose unter portionsweiser oder kontinuierlicher Zudosierung unter Rühren so, daß das Reaktionsgemisch praktisch klar bleibt;
g) unmittelbares Abdestillieren des freiwerdenden Wassers als Butanol/Wasser-Gemisch;
h) es wird vorzugsweise ein leichtes Vakuum von etwa 800 bis 950 mbar während oder nach der Zudosierung der Butanol/Glykose-Mischung angelegt und unter weiterer Wärmezufuhr und Rühren die Abdestillation des Reaktionswassers beendet;
i) anschließend wird der vorgewärmte Fettalkohol in einer Menge von 2 bis 20 Mol pro Mol Glykose, vorzugsweise kontinuierlich zudosiert und gleichzeitig das Butanol im Vakuum abdestilliert;
j) die Abdestillation des Butanols wird so gesteuert, daß 0 bis 30 Mol-% Butylglykosid. bezogen auf 1 Mol der Glykose, im Reaktionsgemisch verbleiben;
k) das Reaktionsgemisch wird auf eine Temperatur unterhalb 95 °C abgekühlt und der saure Katalysator mit einer organischen oder anorganischen basischen Alkali-, Erdalkali- oder Aluminium- bzw. Alkali/Aluminiumverbindung neutralisiert und darüber hinaus auf einen pH-Wert von wenigstens 8, vorzugsweise etwa 9, eingestellt;
l) vorzugsweise wird eine Filtration des Reaktionsgemisches durchgeführt, und zwar vorzugsweise bei einer Temperatur von 80 bis 90 °C;
m) der überschüssige Fettalkohol wird auf eine übliche, das Reaktionsprodukt schonende Weise auf einen Wert von unterhalb 5 Gew.-% abdestilliert.

2. Verfahren nach Anspruch 1, dadurch gekennzeichnet, daß man als Glykose die Glucose, insbesondere in Form der wasserfreien feinteiligen Glucose verwendet.

3. Verfahren nach einem der Ansprüche 1 oder 2, dadurch gekennzeichnet, daß man als sauren Katalysator die Paratoluolsulfonsäure verwendet.

4. Verfahren nach einem der Ansprüche 1 bis 3, dadurch gekennzeichnet, daß man pro Mol Glykose 5 bis 7 Mol des Fettalkohols einsetzt.

5. Verfahren nach einem der Ansprüche 1 bis 4, dadurch gekennzeichnet, daß man die Suspension der Glykose in Butanol fein dispergiert, insbesondere durch Verwendung eines Inline-Mischers.

6. Verfahren nach einem der Ansprüche 1 bis 5, dadurch gekennzeichnet, daß man zur Neutralisation des Katalysators ein Magnesiumalkoholat, insbesondere Magnesiumethylat, oder Zeolith NaA, insbesondere in Mischungen mit Calciumhydroxid, verwendet.

7. Verfahren nach einem der Ansprüche 1 bis 6, dadurch gekennzeichnet, daß man die Abdestillation des Fettalkohols zweistufig durchführt und in einer ersten Stufe in einem Dünnschichtverdampfer eine Abreicherung auf Werte von ca. 40 bis 20 % und in einer zweiten Stufe in einem Kurzwegverdampfer auf einen Endwert von unterhalb 0.5 Gew.-% Fettalkohol, vorzugsweise auf einen Endwert von 3 bis 5 Gew.-% Fettalkohol einstellt.

## Claims

1. A process for the production of surface-active alkyl glycosides containing essentially C₁₂₋₁₈ alkyl or alkenyl radicals by the transacetalization method using butanol, comprising the following steps:
a) butanol is initially introduced into the reaction vessel together with an acidic catalyst;
b) the quantity of butanol is selected so that it amounts to between 4 and 10 mol and preferably to between 6 and 8 mol per mol glycose;
c) part of the butanol, preferably about half, is initially introduced together with the catalyst while the other half is used for suspending the glycose;
d) the catalyst used is a compound showing an acidic reaction, more especially an acid from the group comprising sulfuric acid, phosphoric acid, p-toluene-sulfonic acid and sulfo-acid ion exchanger resins, in a quantity of preferably 0.005 to 0.02 mol per mol of the glycose used;
e) heating the butanol/catalyst mixture to the reflux temperature;
f) adding a preferably preheated suspension of the remaining butanol and the glycose either in portions or continuously with stirring so that the reaction mixture remains substantially clear;
g) directly distilling off the water released in the form of a butanol/water mixture;
h) a light vacuum of approximately 800 to 950 mbar is preferably applied during or after addition of the butanol/glycose mixture and the removal of the water of reaction by distillation is completed with stirring in the presence of more heat;
i) the preheated fatty alcohol is then added, preferably continuously, in a quantity of 2 to 20 mol per mol glycose while, at the same time, the butanol is distilled off *in vacuo*;
j) removal of the butanol by distillation is controlled in such a way that 0 to 30 mol-% butyl glycoside per mol glycose remain in the reaction mixture;
k) the reaction mixture is cooled to a temperature below 95°C and the acidic catalyst is neutralized with an organic or inorganic basic alkali metal, alkaline earth metal or aluminium or alkali metal/aluminium compound and, in addition, is adjusted to a pH value of at least 8 and preferably around 9;
l) the reaction mixture is preferably filtered, preferably at a temperature of 80 to 90°C;
m) the excess fatty alcohol is distilled off to a content below 5% by weight by a standard method which does not affect the reaction product.

2. A process as claimed in claim, characterized in that glucose, more especially in the form of anhydrous fine-particle glucose, is used as the glycose.

3. A process as claimed in claim 1 or 2, characterized in that paratoluene sulfonic acid is used as the acidic catalyst.

4. A process as claimed in any of claims 1 to 3, characterized in that 5 to 7 mol fatty alcohol per mol glycose is used.

5. A process as claimed in any of claims 1 to 4, characterized in that the suspension of the glycose in butanol is finely dispersed, more particularly by using an inline mixer.

6. A process as claimed in any of claims 1 to 5, characterized in that a magnesium alcoholate, more especially magnesium ethylate, or zeolite NaA, more especially in admixture with calcium hydroxide, is used for neutralizing the catalyst.

7. A process as claimed in any of claims 1 to 6, characterized in that removal of the fatty alcohol by distillation is carried out in two stages, in a first stage carried out in a thin-layer evaporator to values of approximately 40 to 20% and, in a second stage carried out in a short-path evaporator, to a final value below 0.5% by weight fatty alcohol and preferably to a final value of 3 to 5% by weight fatty alcohol.

## Revendications

1. Procédé de préparation d'alkylglucosides tensioactifs avec essentiellement des restes alkyle ou alcényle de C₁₂ à C₁₈, qui utilise le procédé de transacétalisation avec le butanol, comprenant les étapes de procédé suivantes :
a) On place le butanol avec un catalyseur acide dans le réacteur,
b) On choisit la quantité de butanol de sorte que, rapportée à 1 mole de glucose, elle est de 4 à 10 moles, de préférence de 6 à 8 moles,
c) On place une partie du butanol, de préférence environ la moitié du butanol avec le catalyseur et on utilise l'autre moitié pour la mise en suspension du glucose,
d) Comme catalyseur, on utilise un composé à réaction acide, en particulier un acide du groupe constitué de l'acide sulfurique, acide phosphorique, acides paratoluènesulfonique et résines échangeuses d'ions sulfacides, en quantité de préférence de 0,005 à 0,02 mole par mole de glucose mis en oeuvre,
e) Chauffage du mélange butanol/catalyseur à température de reflux.
f) Addition d'une suspension de préférence préchauffée du reste de butanol et de glucose avec addition par portion ou en continu sous agitation de sorte, que le mélange réactionnel demeure pratiquement limpide.
g) Elimination immédiate par distillation de l'eau libérée sous forme de mélange butanol-eau,
h) On préfère de préférence un léger vide d'environ 800 à 950 mbars pendant ou après l'addition du mélange butanol/glucose et on termine l'élimination par distillation de l'eau de réaction par apport supplémentaire de calories et agitation,
i) Ensuite, on ajoute l'alcool gras préchauffé à une concentration de 2 à 20 moles par mole de glucose, de préférence en continu et on élimine simultanément le butanol sous vide,
j) On régule l'élimination par distillation du butanol de telle façon qu'il reste dans le mélange réactionnel 0 à 30 % molaires de butylglucoside, rapporté à 1 mole de glucose,
k) On refroidit le mélange réactionnel à une température inférieure à 95 ° C et on neutralise le catalyseur acide avec un composé basique organique ou minéral de métal alcalin, alcalino-terreux ou d'aluminium ou d'alcalin et d'aluminium et ensuite on règle à un pH d'au moins 8, de préférence 9,
l) De préférence, on réalise une filtration du mélange réactionnel et certes, de préférence à une température comprise entre 80 et 90° C.
m) On élimine par distillation l'alcool gras en excès d'une manière usuelle, qui ménage le produit de réaction jusqu'à une concentration de 5 % en poids.

2. Procédé selon la revendication 1, caractérisé en ce qu'on utilise comme sucre le glucose, notamment sous forme de glucose fin anhydre.

3. Procédé selon l'une des revendications 1 ou 2, caractérisé en ce qu'on utilise comme catalyseur acide l'acide paratoluènesulfonique.

4. Procédé selon l'une des revendications 1 à 3, caractérisé en ce qu'on utilise par mole de glucose 5 à 7 moles d'alcool gras.

5. Procédé selon l'une des revendications 1 à 4, caractérisé en ce qu'on disperse finement la suspension de glucose dans le butanol, notamment en utilisant un mélangeur en ligne.

6. Procédé selon l'une des revendications 1 à 5, caractérisé en ce que pour neutraliser le catalyseur, on utilise un alcoolat de magnésium, notamment l'éthylate de magnésium, ou de la zéolite NaA, notamment en mélange avec l'hydroxyde de calcium.

7. Procédé selon l'une des revendications 1 à 6, caractérisé en ce qu'on réalise l'élimination par distillation de l'alcool gras en deux étapes et que dans un premier étage dans un évaporateur à couche mince, on obtient un appauvrissement d'environ 40 à 20 % et dans un deuxième étage dans un évaporateur à distillation moléculaire on règle la concentration finale en alcool gras en dessous de 0,5 % en poids, de préférence à une concentration finale de 3 à 5 % en alcool gras.
